(19) Europäisches Patentamt
European Patent Office
Office européen des brevets

(11) **EP 3 666 854 B1**

(12) **EUROPÄISCHE PATENTSCHRIFT**

(45) Veröffentlichungstag und Bekanntmachung des Hinweises auf die Patenterteilung:
**25.08.2021  Patentblatt 2021/34**

(21) Anmeldenummer: **19213910.3**

(22) Anmeldetag: **05.12.2019**

(51) Int Cl.:
**C07C 2/10** (2006.01)        **C10G 50/00** (2006.01)

(54) **VERFAHREN ZUR OLIGOMERISIERUNG MIT STUFENANGEPASSTEM AUSTAUSCH DES OLIGOMERISIERUNGSKATALYSATORS**

METHOD FOR OLIGOMERISATION WITH STAGE-ADJUSTED EXCHANGE OF OLIGOMERISATION CATALYST

PROCÉDÉ D'OLIGOMÉRISATION À REMPLACEMENT ADAPTÉ AUX ÉTAGES DU CATALYSEUR D'OLIGOMÉRISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2018  EP 18212258**

(43) Veröffentlichungstag der Anmeldung:
**17.06.2020  Patentblatt 2020/25**

(73) Patentinhaber: **Evonik Operations GmbH**
**45128 Essen (DE)**

(72) Erfinder:
• **Peitz, Stephan**
 **45739 Oer-Erkenschwick (DE)**
• **Maschmeyer, Dietrich**
 **45657 Recklinghausen (DE)**
• **Reeker, Helene**
 **44227 Dortmund (DE)**
• **Stochniol, Guido**
 **45721 Haltern am See (DE)**
• **Winterberg, Markus**
 **45731 Waltrop (DE)**
• **Beckmann, Andreas**
 **45657 Recklinghausen (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
EP-A1- 0 395 857        EP-A2- 1 457 475
DE-A1- 19 922 038        DE-A1-102009 027 408

**Beschreibung**

Die vorliegende Erfindung betrifft ein mindestens zweistufiges Verfahren zur Oligomerisierung von kurzkettigen Olefinen in Anwesenheit eines Katalysators, wobei die Regeneration des Katalysators stufenangepasst erfolgt.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich alleine in Deutschland auf mehrere tausend Kilotonnen pro Jahr. Um möglichst hohe Umsätze und einen möglichst kontinuierlichen Betrieb von Oligomerisierungsverfahren zu ermöglichen umfassen industrielle Anlagen meist nicht nur eine, sondern mindestens zwei hintereinandergeschaltete Reaktionsstufen. Dadurch kann das Oligomerisierungsverfahren selbst bei Ausfall einer Reaktionsstufe weiterbetrieben werden. Verfahren zur Oligomerisierung sind beschrieben in DE19922038 und EP1457475.

Die bei der Oligomerisierung von Olefinen eingesetzten Katalysatoren weisen hohe Aktivitäten auf und sind zum Teil an das jeweilige Oligomerisierungsverfahren angepasst. Mit der Zeit verlieren die eingesetzten Katalysatoren jedoch an Aktivität durch Altern und/oder Vergiftung und die Umsätze zu den gewünschten Oligomeren nehmen ab. Deshalb werden Oligomerisierungskatalysatoren üblicherweise in einem Abstand von etwa 4 bis 6 Jahren (48 bis 72 Monate) in allen Reaktionsstufen durch einen frischen Oligomerisierungskatalysator ausgetauscht.

Bei dem bekannten Austausch des Oligomerisierungskatalysators wird zuerst der Reaktor der ersten Reaktionsstufe entleert und frischer Katalysator eingesetzt. Der frische Katalysator kann dabei ein bereits benutzter, aber regenerierter Katalysator oder ein neu hergestellter Katalysator sein. Während des Austauschs des Katalysators in der ersten Reaktionsstufe wird das Einsatzgemisch, welches die zu oligomerisierenden Olefine enthält, um die erste Reaktionsstufe herumgeführt und der oder die Reaktor(en) der dahinterliegenden Reaktionsstufe(n) weiterbetrieben. Nach erfolgreichem Austausch des Katalysatormaterials in der ersten Reaktionsstufe wird diese wieder in Betrieb genommen und direkt im Anschluss der Reaktor/die Reaktoren der zweiten Reaktionsstufe umfahren und deren Katalysator ausgetauscht. Dann folgen in gleicher Weise die optional vorliegenden dritten und ggf. weiteren Reaktionsstufen.

Das hat zur Folge, dass mit einem solchen Austauschkonzept die Anlage für einen langen Zeitraum, nämlich von Beginn bis Ende des Austauschs des Katalysators in jeder einzelnen Reaktionsstufe, mit einer Reaktionsstufe weniger als im Normalbetrieb gefahren wird. Dies führt zu entsprechend lang anhaltenden Umsatzeinbußen. Die Handhabung des Katalysators vor dem Einfüllen in den Reaktor kann außerdem dazu führen, dass die Anfahrzeit, also die Zeit, die der Katalysator braucht, um die gewünschten Umsätze und auch Produktqualitäten zu erzielen, sich verlängert. Zusammen mit den genannten Problemen beim Katalysatoraustausch ergibt sich insgesamt ein erheblicher Umsatzrückgang und das über einen vergleichsweise langen Zeitraum. Auch aus wirtschaftlichen Gründen ist ein solcher Zustand unerwünscht.

Die Aufgabe der vorliegenden Erfindung war die Bereitstellung eines Verfahrens mit mindestens zwei Reaktionsstufen zur Oligomerisierung von Olefinen, welches die vorgenannten Probleme nicht aufweist. Die zugrundeliegende Aufgabe der vorliegenden Erfindung konnte mit dem Verfahren zur Oligomerisierung gemäß Anspruch 1 gelöst werden. Bevorzugte Ausgestaltungen sind in den Unteransprüchen angegeben.

Das erfindungsgemäße Verfahren ist ein Verfahren zur Oligomerisierung von C3- bis C6-Olefinen, bei dem ein Einsatzgemisch, welches die C3- bis C6-Olefine enthält und aus dem als Katalysatorgifte wirkende Stickstoff-, Sauerstoff- und/oder Schwefel-haltige Verunreinigungen zumindest teilweise entfernt worden sind, eingesetzt wird,
wobei die Oligomerisierung in mindestens zwei hintereinanderliegenden Reaktionsstufen, die jeweils aus mindestens einem Reaktor und mindestens einer Destillationskolonne bestehen, unter Verwendung jeweils eines Oligomerisierungskatalysators mit einer Zusammensetzung von 15 bis 40 Gew.-% NiO, 5 bis 30 Gew.-% Al2O3, 55 bis 80 Gew.-% SiO2 und 0,01 bis 2,5 Gew.-% eines Alkalimetalloxids erfolgt, und
wobei zumindest der eine oder die Reaktoren der ersten Reaktionsstufe isotherm betrieben werden, dadurch gekennzeichnet, dass
der Oligomerisierungskatalysator in der ersten Reaktionsstufe spätestens nach 47 Monaten, vorzugsweise spätestens nach 45 Monaten, besonders bevorzugt spätestens nach 43 Monaten durch einen frischen Oligomerisierungskatalysator ausgetauscht wird,
und der Oligomerisierungskatalysator in der oder den dahinerliegenden Reaktionsstufe(n) frühestens nach 48 Monaten bis spätestens nach 72 Monaten durch einen frischen Oligomerisierungskatalysator ausgetauscht wird.

Der Begriff "Reaktionsstufe" umfasst im Sinne der vorliegenden Erfindung aus einem oder mehreren Reaktoren einer oder mehreren Destillationskolonnen. In den Destillationskolonnen werden insbesondere das verbliebene Einsatzgemisch, welches beispielsweise Alkane und nicht umgesetzte Olefine umfasst, von den erzeugten Produktoligomeren getrennt. Übliche verfahrenstechnische Nebenaggregate, die in den Reaktionsstufen eingebaut sein können, wie Vorwärmer für den Feed, Wärmetauscher oder ähnliches, werden hier nicht separat aufgeführt, sondern sind dem Fachmann geläufig.

Der Begriff "frischer Oligomerisierungskatalysator" meint im Sinne der vorliegenden Erfindung sowohl neu hergestellte Katalysatoren als auch regenerierte Katalysatoren, die bereits in einem Reaktor zur Oligomerisierung verwendet worden sind, nach dem Ausbau aber durch die nachfolgend beschriebenen Verfahren regeneriert worden sind.

Das erfindungsgemäße Verfahren bezieht sich hinsichtlich der Angabe der Anzahl der Monate, bei der spätestens oder ab der frühestens ein Austausch des Katalysators in einer bestimmten Reaktionsstufe erfolgt, immer auf den letzten Austausch des Katalysators in der bestimmten Reaktionsstufe. Erfindungsgemäß hat die erste Reaktionsstufe also einen kürzeren Austauschrhythmus als die nachfolgende(n) Reaktionsstufe(n). Der Katalysator der ersten Reaktionsstufe wird also spätestens 47 Monate nach dem Einsetzen dieses Katalysators in die erste Reaktionsstufe gewechselt, die Katalysatoren in der oder den nachfolgenden Reaktionsstufen daher frühestens 48 Monate nach dem Einsetzen dieser Katalysatoren. Der Startzeitpunkt, ab dem die Anzahl der Monate gezählt wird, kann für die verschiedenen Stufen also auch identisch sein, nämlich dann, wenn die Katalysatoren zuletzt gleichzeitig gewechselt worden sind.

Mit dem beschriebenen Ablauf des Austauschens der Oligomerisierungskatalysatoren kann der Zeitraum des Katalysatoraustauschs, in dem ein Umsatzrückgang vorliegt, deutlich verkürzt werden. Es wurde überraschend gefunden, dass der Katalysator in dem Reaktor oder den Reaktoren der ersten Reaktionsstufe schneller altert und/oder schneller vergiftet wird, als die Katalysatoren in der oder den nachfolgenden Reaktionsstufe(n). Wird der Katalysator in dem oder den Reaktoren der ersten Reaktionsstufe gemäß der Erfindung häufiger ausgetauscht als in der oder in den nachfolgenden Reaktionsstufe(n), wird sich der Gesamtzeitraum des Austauschs des Katalysators der dahinterliegenden Stufe(n) verkürzen, da der Katalysator nun in einer Reaktionsstufe weniger ausgetauscht werden muss.

Zwar wird dadurch der addierte Gesamtzeitraum, in dem in allen Reaktionsstufen ein Austausch des Katalysators durchgeführt wird, verlängert, weil im Vergleich zum normalen Austausch ein häufigerer Austausch des Katalysators in dem Reaktor der ersten Reaktionsstufe erfolgt. Dies geht aber damit einher, dass der Austausch der übrigen Katalysatoren im normalen Rhythmus von etwa 48 bis 72 Monaten in allen nachfolgenden Reaktionsstufen gleichzeitig erfolgen kann und daher die Stillstandszeit insgesamt erheblich geringer wird. Außerdem wird durch die erfindungsgemäße Vorgehensweise erreicht, dass die Katalysatoraktivität im Reaktor oder in den Reaktoren der ersten Reaktionsstufe im Vergleich zu einem im normalen Rhythmus von 48 bis 72 Monaten ausgetauschten Katalysator nicht mehr so stark absinkt, da der Zeitraum, die der Katalysator eingesetzt wird und in dem Alterungs- und Vergiftungsprozesse ablaufen können, kürzer ist.

Es hat sich dadurch überraschenderweise gezeigt, dass die Umsatzeinbußen bei dem erfindungsgemäßen Verfahren insgesamt geringer ausgefallen sind, durchschnittlich über einen langen Zeitraum höhere Umsätze ergeben als bei bekannten Verfahren, bei denen die Katalysatoren aller Reaktionsstufen innerhalb der üblichen 48 bis 72 Monate ausgetauscht wird.

Das erfindungsgemäße Verfahren wird ganz allgemein wie folgt durchgeführt. Ausgangspunkt ist ein Verfahren zur Oligomerisierung von Olefinen in mehreren, mindestens zwei Reaktionsstufen. Bei Absinken der Katalysatoraktivität, die beispielsweise über eine GC-Analytik der Reaktionsprodukte beobachtet werden kann, erfolgt zunächst der Austausch des Oligomerisierungskatalysators in der ersten Reaktionsstufe, spätestens nach 36 Monaten ab Einsatz. Dazu wird das Einsatzgemisch, welches die zu oligomerisierenden Olefine enthält, um die erste Reaktionsstufe herumgeführt und direkt zur zweiten Reaktionsstufe geleitet. Aus dem bzw. den nun nicht mehr mit Olefinen beschickten Reaktor(en) der ersten Reaktionsstufe kann der Oligomerisierungskatalysator entnommen werden. Der oder die Reaktor(en) der umfahrenen Reaktionsstufe wird dann zunächst mi einem Inertgas gespült, um flüchtige organische Substanzen zu entfernen. Danach wird der Reaktor geöffnet und der Katalysator entnommen. Sobald der Katalysator aus dem oder den Reaktoren entfernt worden ist, wird dieser unverzüglich mit einem frischen Oligomerisierungskatalysator befüllt. Danach wird der Reaktor oder die Reaktoren angefahren und die Reaktionsstufe nach Abschluss der Anfahrprozedur wieder vollständig als erste Reaktionsstufe geschaltet. Nach 48 Monaten bis spätestens 72 Monaten erfolgt dann der Austausch des Katalysators in der zweiten und eventuell vorhandenen weiteren Reaktionsstufen analog zu der für die erste Reaktionsstufe erklärten Verfahrensweise.

Das erfindungsgemäße Verfahren umfasst mindestens zwei Reaktionsstufen. In einer bevorzugten Ausführungsform umfasst das Verfahren zur Oligomerisierung maximal fünf Reaktionsstufen. Insbesondere bevorzugt ist eine Verfahrensführung mit drei, vier oder fünf Reaktionsstufen. Jede dieser Reaktionsstufen umfasst unabhängig voneinander einen oder mehreren Reaktoren und eine oder mehrere Destillationskolonnen, um die gebildeten Oligomere von dem restlichen Einsatzgemisch abzutrennen. Es ist aber auch denkbar, dass eine der Reaktionsstufen mehrere Reaktoren umfasst, während in einer vorherigen oder nachfolgenden Reaktionsstufe nur ein Reaktor vorhanden ist.

Der eine oder die Reaktoren zumindest der ersten Reaktionsstufe werden isotherm betrieben, werden also in dem Fachmann bekannter Weise, beispielsweise unter Verwendung eines Kühlmediums, gekühlt, um eine möglichst gleichbleibende Temperatur über den oder die Reaktoren zu erhalten. In einer bevorzugten Ausführungsform werden alle Reaktoren in jeder Reaktionsstufe isotherm betrieben. Der oder die Reaktoren in einer der Reaktionsstufen, insbesondere der letzten Reaktionsstufe können aber auch adiabat betrieben werden. Die Formulierung "adiabat betrieben" ist so zu verstehen, dass der oder die Reaktor(en) in der entsprechenden Reaktionsstufe nicht aktiv gekühlt werden. Die bei der Oligomerisierung freiwerdende Wärme wird stattdessen mit dem Produktstrom aus dem Reaktor geführt, wobei in der nachfolgenden Destillationskolonne weniger Energie zur Verdampfung benötigt wird und die Destillation damit energiesparender durchgeführt werden kann.

In einer weiterhin bevorzugten Ausführungsform ist das Volumen des oder der Reaktoren der zweiten und jeder weiteren Reaktionsstufe gleich dem Volumen der vorhergehenden Reaktionsstufe oder ist jeweils kleiner als das der vorhergehenden Reaktionsstufe, da die Menge der Einsatzolefine in den jeweiligen Strömen der zweiten und jeder weiteren Reaktionsstufe typischerweise geringer wird.

Als Olefine für das erfindungsgemäße Verfahren werden C3- bis C6-Olefine, vorzugsweise C3- bis C5-Olefine, besonders bevorzugt C4-Olefine oder darauf basierende Olefingemische, die auch Anteile an analogen Alkanen enthalten können, eingesetzt. Geeignete Olefine sind unter anderem $\alpha$-Olefine, n-Olefine und Cycloalkene, vorzugsweise n-Olefine. In einer bevorzugten Ausführungsform handelt es sich bei dem Olefin um n-Buten.

Die Olefine werden üblicherweise nicht in reiner Form als Edukte eingesetzt, sondern in technisch verfügbaren Mischungen. Der in dieser Erfindung zusätzlich verwendete Begriff Einsatzgemisch ist deshalb so zu verstehend, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem $C_4$-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete Olefingemische sind insbesondere das Raffinat I und das Raffinat II und das Raffinat III.

Es ist bekannt, dass die im erfindungsgemäßen Verfahren eingesetzten Einsatzgemische, die beispielsweise aus Steamcrackern oder katalytischen Crackern stammen, üblicherweise Stickstoff-, Sauerstoff- und/oder Schwefel-haltige Verunreinigungen, die als Katalysatorgifte für die eingesetzten Katalysatoren wirken, im ppm-Bereich enthalten können. Diese Verunreinigungen müssen daher vor der Oligomerisierung aus dem die zu oligomerisierenden Olefine enthaltenden Einsatzgemisch entfernt werden. Das im erfindungsgemäßen Verfahren verwendete Einsatzgemisch ist demnach ein Einsatzgemisch, aus dem als Katalysatorgifte wirkende Stickstoff-, Sauerstoff- und/oder Schwefel-haltige Verunreinigungen zumindest teilweise entfernt worden sind.

Die Entfernung von Stickstoff-, Sauerstoff- und/oder Schwefel-haltige Verunreinigungen aus Kohlenwasserstoffströmen ist dem Fachmann bekannt. Die als Einsatzgemisch verwendeten Kohlenwasserstoffströme können dazu beispielsweise über ein oder mehrere Schutzbetten ("guard beds"), in denen ein Material vorhanden ist, dass die Verunreinigungen aufnimmt bzw. adsorbiert, geleitet werden. Möglich ist zum Teil auch eine destillative Abtrennung von leicht- oder schwersiedenden Verunreinigungen in vorgelagerten Leichtsieder- oder Schwersiederkolonnen. Die Verunreinigungen sollen damit so ausreichend aus den Kohlenwasserstoffströmen entfernt werden, dass eine Vergiftung des Katalysators in der Oligomerisierung verzögert oder vollständig verhindert wird.

Der eine oder die Reaktoren der einzelnen Reaktionsstufen enthalten jeweils einen Oligomerisierungskatalysator mit einer Zusammensetzung von 15 bis 40 Gew.-%, vorzugsweise 15 bis 30 Gew.-% NiO, 5 bis 30 Gew.-% $Al_2O_3$, 55 bis 80 Gew.-% $SiO_2$ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. In einer besonders bevorzugten Ausführungsform der vorliegenden Erfindung ist der Oligomerisierungskatalysator im Wesentlichen frei von Titandioxid und/oder Zirkoniumdioxid, insbesondere enthält der Oligomerisierungskatalysator weniger als 0,5 Gew.-

%, bevorzugt weniger als 0,1 Gew.-%, besonders bevorzugt weniger als 0,01 Gew.-% Titandioxid und/oder Zirkoniumdioxid in seiner Gesamtzusammensetzung. Der Oligomerisierungskatalysator liegt dabei insbesondere in Form eines Granulats, eines Extrudats oder in Tablettenform vor.

Die (heterogenen) Oligomerisierungskatalysatoren in den einzelnen Reaktoren der Reaktionsstufen können jeweils unabhängig voneinander Oligomerisierungskatalysatoren mit der oben genannten Zusammensetzung ausgewählt werden.

Der erfindungsgemäße Oligomerisierungskatalysator umfasst grundsätzlich Nickeloxid auf einem Alumosilicat-Trägermaterial. Das Trägermaterial kann ein amorphes, mesoporöses Alumosilicat, ein kristallines, mikroporöses Alumosilicat oder eine Alumosilicat, welches amorphe und kristalline Phasen aufweist sein. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Silica-Alumina-Supportmaterial kleine kristalline Domänen aufweist.

Der Oligomerisierungskatalysator weist vorzugsweise eine spezifische Oberfläche (berechnet nach BET) von 150 bis 700 $m^2$/g, weiterhin bevorzugt von 190 bis 600 $m^2$/g, besonders bevorzugt von 220 bis 550 $m^2$/g aufweisen. Die BET-Oberfläche wird mittels Stickstoff-Physisorption gemäß DIN-ISO 9277 (Stand: 2014-01) gemessen.

Die in den einzelnen Reaktoren in den Reaktionsstufen vorhandenen Oligomerisierungskatalysatoren können jeweils unabhängig voneinander aus den vorgenannten Substanzen ausgewählt werden. Die einzelnen Oligomerisierungskatalysatoren in den Reaktoren sind dabei nicht immer exakt identisch, sondern unterscheiden sich in der Zusammensetzung voneinander, möglicherweise auch nur in geringem Umfang. Dies liegt auch daran, dass selbst wenn zum Zeitpunkt der ersten Inbetriebnahme des erfindungsgemäßen Verfahrens jeder Reaktor eine völlig identische Katalysatorzusammensetzung enthält, sich diese Zusammensetzung während des Betriebs mit der Zeit durch verschiedenste Effekte im Laufe der Jahre (Regenerierter Katalysator verhalten sich anders als neu hergestellte Katalysatoren, Abrieb während des Betriebes, unterschiedlich schnelle Alterung und/oder Vergiftung, etc.) ändern.

Mit steigender Einsatzzeit des Oligomerisierungskatalysators bei der Oligomerisierung kann es zu einer Abnahme des Umsatzes und/oder der Selektivität (allgemein der Katalysatoraktivität) durch Altern und/oder Vergiftung kommen. Der erfindungsgemäße Katalysator wird wie erfindungsgemäß beschrieben ausgetauscht und durch einen frischen Oligomerisierungskatalysator, also einen neuen oder einen regenerierten Oligomerisierungskatalysator, ersetzt.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden.

Nach dem Einsatz des hergestellten Oligomerisierungskatalysators in der Oligomerisierung kann der Katalysator auch regeneriert werden, um die Katalysatoraktivität (für die Oligomerisierung) wieder zu steigern. Die Regenerierung des Oligomerisierungskatalysators umfasst insbesondere die Schritte A) des Abbrennens und B) des Imprägnierens mit einer Lösung einer Übergangsmetallverbindung. Dabei sollten das Übergangsmetall in dem zu regenerierenden Katalysator und das Übergangsmetall in der Imprägnierlösung identisch sein.

Es ist möglich, dass der Oligomerisierungskatalysator nach seinem Einsatz in der Oligomerisierung Ablagerungen organischer Stoffe aufweist, die entfernt werden müssen. Die Entfernung der im Katalysator abgelagerten organischen Verbindungen geschieht vorzugsweise in Schritt A) durch einfaches Abbrennen, wodurch Kohlenstoffoxide und Wasser entstehen. Das Abbrennen in Schritt A) kann in einem Ofen, beispielsweise in einem Drehrohrofen oder einem Schachtofen, kontinuierlich oder diskontinuierlich durchgeführt werden. Dazu wird der Oligomerisierungskatalysators, beispielsweise in Form eines Granulats, dem Ofen zugeführt und vorzugsweise bei einer Ofentemperatur von 400 bis 600 °C, besonders bevorzugt von 500 bis 600 °C abgebrannt. Das Abbrennen kann unter Einsatz von zusätzlich zugeleiteter Verbrennungsluft durchgeführt werden. Diese Verbrennungsluft kann im Gegenstrom zugeführt werden, optional wird zusätzlich weitere Luft über geeignete Einlässe in den Oligomerisierungskatalysator eingeblasen werden, um einen schnellen Abbrand zu gewährleisten.

Schritt B) umfasst als Schritt B1) eine Beaufschlagung/Imprägnierung des in Schritt A) abgebrannten Oligomerisierungskatalysators mit einer Lösung einer Übergangsmetallverbindung, insbesondere einer Lösung einer Nickelverbindung und als Schritt B2) eine Kalzination des beaufschlagten Oligomerisierungskatalysators.

Im Folgenden wird die Durchführung des Schritts B1) der Regeneration am Beispiel von Nickel diskutiert. Die Aussagen sind aber auch auf andere Übergangsmetall übertragbar.

Die Imprägnierung mit Nickel in Schritt B1) kann ähnlich wie die Herstellung des Oligomerisierungskatalysators erfolgen, optional jedoch mit dem Unterschied, dass eine Nickellösung mit einer geringeren Nickelkonzentration als bei der Herstellung des Oligomerisierungskatalysators verwendet werden kann. Prinzipiell kann dazu jede lösliche Nickelverbindung wie Nickelnitrat ($Ni(NO_3)_2$), Nickelacetat ($Ni(ac)_2$), Nickelacetylacetonat ($Ni(acac)_2$), Nickelsulfat ($NiSO_4$) oder Nickelcarbonat ($NiCO_3$) zur Herstellung einer wässrigen oder ammoniakalischen Nickel-Lösung verwendet werden.

Als besonders vorteilhaft hat sich die Verwendung von NiHAC-Lösungen erwiesen, die durch Auflösen von

Nickelcarbonat ($NiCO_3$) in konzentrierten Ammoniaklösungen ggf. mit Zusatz von Ammoniumcarbonat erhältlich sind. Derartige Lösungen können mit Nickelgehalten von 0,5 bis 14 Gew.-%, insbesondere von 2 bis 10 Gew.-%, ganz besonders bevorzugt von 4 bis 8 Gew.-% für die Imprägnierung verwendet werden.

Zum Nickelauftrag wird der in Schritt A) abgebrannte Oligomerisierungskatalysator beispielsweise mit einer NiHAC-Lösung mit Nickelgehalten von 0,5 bis 14 Gew.-%, insbesondere von 2 bis 10 Gew.-%, ganz besonders von 4 bis 8 Gew.-% bis zur Sättigung der im Oligomerisierungskatalysator vorhandenen Poren imprägniert. Die Imprägnierung kann mit einem dem Fachmann geläufigen Verfahren wie beispielsweise durch Besprühen bis zum permanenten Auftreten eines Flüssigkeitsfilmes auf der Oberfläche (incipient wetness) durchgeführt werden. Beträgt die Lösungsaufnahme etwa 0,8 bis 1,2 g Lösung pro g Oligomerisierungskatalysator, kann man erreichen, dass etwa 0,5 bis 6 Gew.-% an zusätzlichem Nickel in Form eines basischen Carbonates abgeschieden werden.

Nach dem Schritt B1) kann der imprägnierte Oligomerisierungskatalysator in einer geeigneten Trocknungsapparatur, beispielsweise einem Bandtrockner mit Luftstrom oder auch einem Konustrockner, bei Temperaturen zwischen 100 und 250 °C, vorzugsweise zwischen 120 und 220 °C und Normaldruck oder auch im Vakuum getrocknet werden bevor die Kalzination in Schritt B2) erfolgt. Durch die Trocknung wird insbesondere Wasser oder überschüssigen Ammoniak aus der NiHAC-Lösung ausgetragen.

Die Kalzination des Oligomerisierungskatalysators in Schritt B2) kann in einem geeigneten Ofen, wie beispielsweise einem Schachtofen oder Drehrohrofen, kontinuierlich oder diskontinuierlich durchgeführt werden. Bei einer kontinuierlichen Kalzination wird weiterhin bevorzugt ein Gas im Gegenstrom durch den Oligomerisierungskatalysator, insbesondere in Form eines Granulats geleitet. Als Gas kann Luft, Stickstoff oder eine Mischung davon verwendet werden. Der Gasstrom kann 0,2 bis 4 $m^3$ Gas pro kg Granulat und Stunde und die Eintrittstemperatur des Gases von 400 bis 800 °C, vorzugsweise 450 bis 700 °C betragen. Zusätzlich zu dieser über das Gas eingetragenen Wärme kann Energie durch aktives Heizen der Wände des Ofens eingetragen werden.

Die Kalzinationstemperatur in dem Ofen kann in Schritt B2) 400 bis 800 °C, vorzugsweise 450 bis 700 °C, besonders bevorzugt 500 bis 600 °C betragen. Diese Temperatur kann über mehrere Stunden, vorzugsweise 5 bis 60 Stunden, besonders bevorzugt 10 bis 40 Stunden gehalten werden, bevor der Katalysator abgekühlt wird. Das Abkühlen findet vorzugsweise im Stickstoffstrom statt. Dem Stickstoff kann zusätzlich Luft hinzugefügt werden, wobei die Luftmenge vorzugsweise kontrolliert werden sollte. Die Menge an Luft, die dem Stickstoff vorzugsweise hinzugefügt wird, kann 100 bis 10000 Vol.-ppm, bevorzugt 300 bis 7000 Vol.-ppm betragen.

Die Oligomerisierung des erfindungsgemäßen Verfahren kann bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55 bar durchgeführt werden. In einer bevorzugten Ausführungsform der vorliegenden Erfindung wird die Oligomerisierung in flüssiger Phase durchgeführt. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt.

Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, besonders bevorzugt zwischen 3 $h^{-1}$ und 25 $h^{-1}$.

In einer Ausführungsform, insbesondere bei Verwendung eines Katalysators, welcher eine Nickelverbindung, vorzugsweise Nickeloxid, auf einem Alumosilicat-Trägermaterial umfasst, beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach der folgendenden allgemeinen Formel, wobei sich der Anteil der einzelnen Dimerfraktionen auf die Gesamtdimer-Fraktion bezieht:

$$\frac{\text{(einfach verzweigte Dimere (Gew.-\%)} + 2 \text{ x zweifach verzweigte Dimere (Gew.-\%))}}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das $C_9$-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

Beispiele

Beispiel 1 (nicht erfindungsgemäß):

In einer 3-stufigen Oligomerisierungsanlage werden die Katalysatorfüllungen jeder Stufe typischerweise alle 5 Jahre nacheinander gewechselt. Pro Stufe dauert ein solcher Wechselvorgang etwa 21 Tage. Betrachtet wird hier ein Zeitraum von 15 Jahren, wo demnach 3 Abstellungen erfolgen, um den Katalysator zu wechseln.

Die 1. Stufe produziert in den ersten 3 Jahren mit frischem Katalysator im Mittel 570 Tonnen Oligomerisat pro Tag. Nach einer gewissen Standzeit des Katalysators in dem Oligomerisierungsreaktor sinkt die produzierte Menge an Oligomerisat ab. Die erste Stufe kann nach 3 Jahren, also im 4. und 5. Jahr, dann nur noch 500 Tonnen Oligomerisat pro Tag produzieren.

Der Aktivitätsverlust in der 2. und 3. Reaktionsstufe fällt deutlich geringer aus und wird hier vernachlässigt. Die 2. Stufe produziert während der 5 Jahre im Mittel 285 Tonnen Oligomerisat pro Tag. Die 3. Stufe produziert während der 5 Jahre im Mittel 95 Tonnen Oligomerisat pro Tag.

Durch die 3 Abstellungen innerhalb der 15 Jahre und die reduzierte Aktivität der 1. Stufe in jeweils 2 Jahren vor dem Austausch des Katalysators entfallen insgesamt etwa 208,7 Kilotonnen Oligomerisat.

Beispiel 2 (erfindungsgemäß):

In einer 3-stufigen Oligomerisierungsanlage werden die Katalysatorfüllungen der 1. Stufe alle 3 Jahre, die der 2. und 3. Stufe alle 5 Jahre gewechselt. Pro Stufe dauert ein solcher Wechselvorgang 21 Tage. Betrachtet wird hier ein Zeitraum von 15 Jahren, wo demnach 5 Abstellungen der 1. Reaktionsstufe und 3 Abstellungen der 2. und 3. Reaktionsstufe erfolgen, um den Katalysator zu wechseln

Die 1. Stufe produziert in den 3 Jahren im Mittel 570 Tonnen Oligomerisat pro Tag. Vor einem deutlichen Absinken der Aktivität wird der Kat ausgetaucht. Die 2. Stufe produziert während der 5 Jahre im Mittel 285 Tonnen Oligomerisat pro Tag. Die 3. Stufe produziert während der 5 Jahre im Mittel 95 Tonnen Oligomerisat pro Tag.

Durch die 5 Abstellungen der 1. Reaktionsstufe und die 3 Abstellungen in den letzten beiden Stufen innerhalb der 15 Jahre entfallen insgesamt 83,8 Kilotonnen Oligomerisat.

Die entfallene Menge an Oligomerisat ist bei dem erfindungsgemäßen Wechselzyklus also deutlich geringer. Das häufigere Wechseln des Katalysators der 1. Reaktionsstufe ermöglicht es somit, den Aktivitätsverlust und die damit einhergehenden Mindermenge an Oligomerisat im Vergleich zum nicht erfindungsgemäßen Wechselzyklus überzukompensieren.

**Patentansprüche**

1. Verfahren zur Oligomerisierung von C3- bis C6-Olefinen, bei dem ein Einsatzgemisch, welches die C3- bis C6-Olefine enthält und aus dem als Katalysatorgifte wirkende Stickstoff-, Sauerstoff- und/oder Schwefel-haltige Verunreinigungen zumindest teilweise entfernt worden sind, eingesetzt wird,
wobei die Oligomerisierung in mindestens zwei hintereinanderliegenden Reaktionsstufen, die jeweils aus mindestens einem Reaktor und mindestens einer Destillationskolonne bestehen, unter Verwendung jeweils eines Oligomerisierungskatalysators mit einer Zusammensetzung von 15 bis 40 Gew.-% NiO, 5 bis 30 Gew.-% $Al_2O_3$, 55 bis 80 Gew.-% $SiO_2$ und 0,01 bis 2,5 Gew.-% eines Alkalimetalloxids erfolgt, und
wobei zumindest der eine oder die Reaktoren der ersten Reaktionsstufe isotherm betrieben werden, **dadurch gekennzeichnet, dass**
der Oligomerisierungskatalysator in der ersten Reaktionsstufe spätestens nach 47 Monaten, vorzugsweise spätestens nach 45 Monaten, besonders bevorzugt spätestens nach 43 Monaten durch einen frischen Oligomerisierungskatalysator ausgetauscht wird,

und der Oligomerisierungskatalysator in der oder den dahinerliegenden Reaktionsstufe(n) frühestens nach 48 Monaten bis spätestens nach 72 Monaten durch einen frischen Oligomerisierungskatalysator ausgetauscht wird.

**2.** Verfahren zur Oligomerisierung nach Anspruch 1, wobei der der Oligomerisierungskatalysator in der ersten Reaktionsstufe frühestens nach 6 Monaten durch einen frischen Oligomerisierungskatalysator ausgetauscht wird.

**3.** Verfahren zur Oligomerisierung Anspruch 1 oder 2, wobei das Verfahren drei, vier oder fünf Reaktionsstufen umfasst.

**4.** Verfahren zur Oligomerisierung nach Anspruch 3, wobei der Austausch des Katalysators der zweiten und allen nachfolgenden Reaktionsstufen gleichzeitig erfolgt.

**5.** Verfahren zur Oligomerisierung nach einem der Ansprüche 1 bis 4, wobei der oder die Reaktor(en) in jeder Reaktionsstufe isotherm betrieben werden.

**6.** Verfahren zur Oligomerisierung nach einem der Ansprüche 1 bis 5, wobei das Volumen des oder der Reaktoren der zweiten und jeder weiteren Reaktionsstufe gleich dem Volumen der vorhergehenden Reaktionsstufe ist oder jeweils kleiner ist als das der vorhergehenden Reaktionsstufe.

**7.** Verfahren zur Oligomerisierung nach einem der Ansprüche 1 bis 6, wobei der frische Oligomerisierungskatalysator ein neu hergestellter Oligomerisierungskatalysator oder ein regenerierter Oligomerisierungskatalysator ist.

**8.** Verfahren zur Oligomerisierung nach Anspruch 7, wobei der regenerierte Oligomerisierungskatalysator durch die Schritte A) Abbrennen eines für die Oligomerisierung benutzten Oligomerisierungskatalysators und B) Imprägnieren des in Schritt A) abgebrannten Oligomerisierungskatalysators mit einer Lösung einer Übergangsmetallverbindung und anschließender Kalzination hergestellt wird.

**9.** Verfahren zur Oligomerisierung nach Anspruch 8, wobei in Schritt B) der imprägnierte Oligomerisierungskatalysator vor der Kalzination bei Temperaturen zwischen 100 und 250 °C und bei Normaldruck oder im Vakuum getrocknet wird.

**10.** Verfahren zur Oligomerisierung nach einem der Ansprüche 1 bis 9, wobei die Oligomerisierung bei einer Temperatur im Bereich von 50 bis 200 °C und bei einem Druck von 10 bis 70 bar durchgeführt wird.

**Claims**

**1.** Process for the oligomerization of C3- to C6-olefins, in which a starting mixture is used comprising the C3- to C6-olefins and from which nitrogen-, oxygen- and/or sulfur-containing impurities that act as catalyst poisons have been at least partially removed,
wherein the oligomerization is carried out in at least two successive reaction stages, which each consist of at least one reactor and at least one distillation column, in each case using an oligomerization catalyst having a composition of 15 to 40% by weight NiO, 5 to 30% by weight $Al_2O_3$, 55 to 80% by weight $SiO_2$ and 0.01 to 2.5% by weight of an alkali metal oxide, and
wherein at least the reactor(s) of the first reaction stage are operated isothermally, **characterized in that** the oligomerization catalyst in the first reaction stage is exchanged for a fresh oligomerization catalyst at the latest after 47 months, preferably at the latest after 45 months, particularly preferably at the latest after 43 months,
and the oligomerization catalyst in the following reaction stage(s) is exchanged for a fresh oligomerization catalyst at the earliest after 48 months to at the latest after 72 months.

**2.** Process for the oligomerization according to Claim 1, wherein the oligomerization catalyst in the first reaction stage is exchanged for a fresh oligomerization catalyst at the earliest after 6 months.

**3.** Process for the oligomerization according to Claim 1 or 2, wherein the process comprises three, four or five reaction stages.

**4.** Process for the oligomerization according to Claim 3, wherein the catalyst of the second and all subsequent reaction stages are exchanged at the same time.

**5.** Process for the oligomerization according to any of Claims 1 to 4, wherein the reactor (s) in each reaction stage are operated isothermally.

6. Process for the oligomerization according to any of Claims 1 to 5, wherein the volume of the reactor (s) of the second and each further reaction stage is equal to the volume of the preceding reaction stage or is in each case smaller than that of the preceding reaction stage.

7. Process for the oligomerization according to any of Claims 1 to 6, wherein the fresh oligomerization catalyst is a newly produced oligomerization catalyst or a regenerated oligomerization catalyst.

8. Process for the oligomerization according to Claim 7, wherein the regenerated oligomerization catalyst is produced by step A), burning off of an oligomerization catalyst used for the oligomerization, and B) impregnating the oligomerization catalyst burnt off in step A) with a solution of a transition metal compound and subsequent calcination.

9. Process for the oligomerization according to Claim 8, wherein in step B) the impregnated oligomerization catalyst is dried at temperatures between 100 and 250°C and at standard pressure or under reduced pressure prior to the calcination.

10. Process for the oligomerization according to any of Claims 1 to 9, wherein the oligomerization is carried out at a temperature in the range of 50 to 200°C and at a pressure of 10 to 70 bar.

**Revendications**

1. Procédé pour l'oligomérisation d'oléfines en C3 à C6, dans lequel un mélange d'alimentation est utilisé, qui contient les oléfines en C3 à C6 et duquel des impuretés contenant de l'azote, de l'oxygène et/ou du soufre agissant comme poisons de catalyseur ont été au moins partiellement éliminées,

   l'oligomérisation étant réalisée dans au moins deux stades de réaction consécutifs, qui sont constitués à chaque fois d'au moins un réacteur et d'au moins une colonne de distillation, en utilisant à chaque fois un catalyseur d'oligomérisation doté d'une composition de 15 à 40 % en poids de NiO, 5 à 30 % en poids de $Al_2O_3$, 55 à 80 % en poids de $SiO_2$ et 0,01 à 2,5 % en poids d'un oxyde de métal alcalin, et

   l'au moins un ou les réacteurs du premier stade de réaction fonctionnant de manière isotherme,

   **caractérisé en ce que**

   le catalyseur d'oligomérisation dans le premier stade de réaction est remplacé par un catalyseur d'oligomérisation frais au plus tard après 47 mois, de préférence au plus tard après 45 mois, particulièrement préférablement au plus tard après 43 mois,

   et le catalyseur d'oligomérisation dans le stade de réaction ou les stades de réaction subséquent(s) est remplacé par un catalyseur d'oligomérisation frais au plus tôt après 48 mois jusqu'à au plus tard après 72 mois.

2. Procédé pour l'oligomérisation selon la revendication 1, le catalyseur d'oligomérisation dans le premier stade de réaction étant remplacé par un catalyseur d'oligomérisation frais au plus tôt après 6 mois.

3. Procédé pour l'oligomérisation selon la revendication 1 ou 2, le procédé comprenant trois, quatre ou cinq stades de réaction.

4. Procédé pour l'oligomérisation selon la revendication 3, le remplacement du catalyseur du deuxième stade de réaction et de tous les autres stades de réaction suivants étant réalisé simultanément.

5. Procédé pour l'oligomérisation selon l'une quelconque des revendications 1 à 4, le ou les réacteur(s) fonctionnant de manière isotherme dans chaque stade de réaction.

6. Procédé pour l'oligomérisation selon l'une quelconque des revendications 1 à 5, le volume du ou des réacteurs du deuxième stade de réaction et de chaque autre stade de réaction étant égal au volume du stade de réaction précédent ou étant à chaque fois plus petit que celui du stade de réaction précédent.

7. Procédé pour l'oligomérisation selon l'une quelconque des revendications 1 à 6, le catalyseur d'oligomérisation frais étant un catalyseur d'oligomérisation nouvellement préparé ou un catalyseur d'oligomérisation régénéré.

8. Procédé pour l'oligomérisation selon la revendication 7, le catalyseur d'oligomérisation régénéré étant préparé par les étapes A) de brûlage d'un catalyseur d'oligomérisation utilisé pour l'oligomérisation et B) d'imprégnation du catalyseur d'oligomérisation brûlé dans l'étape A) avec une solution d'un composé de métal de transition et de calcination ultérieure.

9. Procédé pour l'oligomérisation selon la revendication 8, dans lequel dans l'étape B), le catalyseur d'oligomérisation imprégné est séché, avant la calcination, à des températures comprises entre 100 et 250 °C et à pression normale ou sous vide.

10. Procédé pour l'oligomérisation selon l'une quelconque des revendications 1 à 9, l'oligomérisation étant mise en œuvre à une température dans la plage de 50 à 200 °C et à une pression de 10 à 70 bars.

**IN DER BESCHREIBUNG AUFGEFÜHRTE DOKUMENTE**

**In der Beschreibung aufgeführte Patentdokumente**

- DE 19922038
- EP 1457475 A